Europäisches Patentamt

European Patent Office

Office européen des brevets

(19)

(11) Numéro de publication : **0 351 311 B1**

(12) **FASCICULE DE BREVET EUROPEEN**

(45) Date de publication du fascicule du brevet :
30.10.91 Bulletin 91/44

(51) Int. Cl.$^5$ : **B01J 29/04**, C07C 2/00,
C07C 15/00

(21) Numéro de dépôt : 89401993.4

(22) Date de dépôt : 11.07.89

(54) **Catalyseur du type gallosilicate et son utilisation en aromatisation des gaz légers C2-C4.**

(30) Priorité : 12.07.88 FR 8809631

(43) Date de publication de la demande :
17.01.90 Bulletin 90/03

(45) Mention de la délivrance du brevet :
30.10.91 Bulletin 91/44

(84) Etats contractants désignés :
BE DE GB IT NL

(56) Documents cités :
EP-A- 0 269 503
EP-A- 0 303 527

(73) Titulaire : INSTITUT FRANCAIS DU PETROLE
4, Avenue de Bois-Préau
F-92502 Rueil-Malmaison (FR)

(72) Inventeur : Petit, Laurent
10 rue Alibert
F-75010 Paris (FR)
Inventeur : Bournonville, Jean-Paul
43, rue des Groues Vauréal
F-95000 Cergy Pontoise (FR)
Inventeur : Guth, Jean-Louis
59, rue Bellevue Brunstatt
F-68200 Mulhouse (FR)
Inventeur : Raatz, Francis
10, Allée Jacques Prévert
F-78260 Achères (FR)
Inventeur : Seive, Alain
21, rue du Manège
F-68100 Mulhouse (FR)

## Description

La présente invention concerne :
— un catalyseur à base d'une matrice et d'une zéolithe de type MFI contenant du silicium et du gallium synthétisée en milieu fluorure et,
— l'utilisation de ces catalyseurs dans les réactions d'aromatisation des gaz légers $C_2$-$C_4$ en présence ou non d'oléfines.

Une demande parallèle EP-A-89401994.2 à été déposée le même jour sur des catalyseurs similaires.

La synthèse dans des milieux fluorures de ce type de zéolithe de structure MFI consiste :

a) dans une première étape à préparer un mélange réactionnel en solution à un pH inférieur ou égal à environ 10 comprenant, notamment de l'eau, au moins une source de silicium, une source de gallium trivalent, au moins une source d'au moins un ion fluorure $F^-$ et une source d'au moins un agent structurant fournissant des cations organiques contenant de l'azote. L'agent structurant est choisi parmi les di-, trialkylamines et les cations ammonium dérivant par protonation des dites amines, et/ou les cations tétraalkylammonium, les groupes alkyl étant de préférence les groupes n-propyl. Ledit mélange a une composition en termes de rapports molaires comprise dans les intervalles suivants :

$Si^{IV}/Ga^{III}$ : 2-1000

$F^-/Si^{IV}$ : 0,05-3

structurant organique/$Si^{IV}$ : 0,04-1

$H_2O/Si^{IV}$ : 4-400,

b) dans une deuxième étape à chauffer le dit mélange à une température au plus égale à environ 270°C, de manière avantageuse entre 80 et 220°C et de préférence entre 140 et 210°C, pendant une durée suffisante pour obtenir des cristaux de gallosilicate,

c) dans une troisième étape à calciner les dits cristaux à une température supérieure à 400°C et de préférence comprise entre 500 et 600°C. L'étape de calcination a pour but l'élimination des cations organiques ou ammonium contenus dans le solide brut de synthèse.

La composition préférentiellement choisie du mélange réactionnel de départ, en solution aqueuse, est caractérisée par des rapports molaires compris dans les intervalles suivants :

$Si^{IV}/Ga^{III}$ : 8-1000

$F^-/Si^{IV}$ : 0,2-2

structurant organique/$Si^{IV}$ : 0,06-0,75

$H_2O/Si^{IV}$ : 6-200.

Les sources de l'élément $Si^{IV}$ utilisées dans la formation du milieu réactionnel sont, par exemple :
— les silices sous forme d'hydrogels, d'aérogels, de suspensions colloïdales,
— les silices résultant de la précipitation de solutions de silicates solubles, ou de l'hydrolyse d'esters siliciques comme l'ester tétraéthylique de l'acide monoorthosilicique $Si(OC_2H_5)_4$ ou de complexes comme le fluorosilicate d'ammonium $(NH_4)_2SiF_6$, ou de sodium $Na_2SiF_6$,
— les silices préparées par des traitements d'extraction et d'activation de composés cristallisés naturels ou synthétiques.

Les sources de l'élément $Ga^{III}$ utilisées sont, par exemple :
— les sels de gallium (sulfate, nitrate, chlorure, fluorure, acétate par exemple),
— les hydroxydes, les hydroxyoxydes et oxydes de gallium, les gallates et différents esters.

Il est également possible d'utiliser les sources contenant les éléments silicium et gallium associés tels par exemple des verres ou des co-gels.

Les sources des éléments silicium et gallium peuvent être engagées sous forme de fluides ou de solides pulvérulents, mais également sous forme d'agglomérats, tels que par exemple pastilles ou extrudés, pouvant être transformés en zéolithes sans modification de la forme.

Les structurants organiques utilisés sont, par exemple :
— les dialkylamines et les trialkylamines qui seront ensuite transformées in situ en cations lors de l'ajustement du pH à une valeur inférieure à 10,
— les cations tétraalkylammonium ajoutés sous forme d'un de leurs sels comme par exemple le bromure de tétrapropylammonium.

Les groupes alkyl sont de préférence les groupes n-propyl.

Les ions fluorures sont ajoutés sous forme d'acide fluorhydrique ou de fluorures minéraux ou organiques, comme par exemple le fluorure de sodium, le fluorure d'ammonium $NH_4F$, le bifluorure d'ammonium $NH_4HF_2$, le fluorure de tétrapropylammonium $(C_3H_7)_4NF$, ou de composés hydrolysables pouvant libérer au moins un ion fluorure dans l'eau, comme par exemple $SiF_4$ ou $(NH_4)_2SiF_6$. l'acide fluorhydrique, le fluorure d'ammonium ou le bifluorure d'ammonium sont des produits préférés car ils sont peu onéreux et ils permettent d'obtenir des

2

zéolithes de type gallosilicate protonées par simple calcination de la zéolithe résultant de la synthèse.

Le pH du milieu réactionnel est inférieur à environ 10, avantageusement compris entre 2 et 10 et de manière plus préférée entre 4 et 8. Il peut être obtenu soit directement à partir de l'un ou plusieurs des produits composant le milieu réactionnel, soit par l'ajout audit milieu d'un acide, d'une base, d'un sel d'acide d'un sel basique ou d'un mélange tampon complémentaire.

L'ajout de cristaux (germes) au mélange réactionnel et l'agitation facilitent généralement la cristallisation et ont également une influence sur la taille des cristaux de zéolithes formées.

Le chauffage du mélange réactionnel est fait de préférence dans un autoclave revêtu intérieurement de polytétrafluoroéthylène (PTFE). Suivant la composition, l'ajout de germes, la température et l'agitation ou non, la durée de chauffage se situe généralement entre 6 et 650 heures. Lorsque la cristallisation est achevée, le solide obtenu est filtré et lavé à l'eau désionisée.

Les zéolithes de type gallosilicate obtenues par le procédé selon l'invention sont identifiées de manière commode à partir de leur diagramme de diffraction des rayons X. Il peut être obtenu à l'aide d'un diffractomètre en utilisant la méthode des poudres avec le rayonnement K alpha du cuivre. Les équidistances réticulaires $d_{hkl}$ caractéristiques de l'échantillon sont calculées par la relation de Bragg, à partir de la position des pics de diffraction, représentée par l'angle 2 thêta. L'estimation de l'erreur de mesure $d_{hkl}$ se calcule en fonction de l'erreur absolue 2 thêta affectée à la mesure de 2 thêta par la relation de Bragg. L'erreur absolue 2thêta, couramment admise, est égale à ± 0,2°. L'intensité relative I/Io, I étant l'intensité d'une raie donnée et Io l'intensité de la raie la plus forte, affectée à chaque valeur de $d_{hkl}$ est estimée à partir de la hauteur du pic de diffraction correspondant. Les tableaux 1 et 2 ci-après représentent les diagrammes de diffraction des rayons X caractéristiques de deux zéolithes de type gallosilicate A et B obtenues selon le procédé de la présente invention, calcinées à 550°C, sous air, le rapport molaire $Si^{IV}/Ga^{III}$ de A est au plus égal à 50, le rapport molaire $Si^{IV}/Ga^{III}$ de B est au moins égal à 50. Les colonnes de $d_{hkl}$ des tableaux 1 et 2 représentent les valeurs extrêmes que peuvent prendre les différentes équidistances $d_{hkl}$. Les valeurs dépendent du rapport $Si^{IV}/Ga^{III}$ et de la nature des cations de compensation, chacune des valeurs indiquées dans les tableaux doit encore être affectée de l'erreur de mesure $d_{hkl}$. Pour caractériser les intensités relatives I/Io, une échelle de symbole est souvent utilisée : FF = très fort, F = fort, mF = moyen à fort, m = moyen, mf = moyen à faible, f = faible, ff = très faible. Ces intensités relatives dépendent également en partie de la composition des zéolithes de type gallosilicate obtenues.

Les solides obtenus par la procédure de synthèse décrite précédemment sont des zéolithes de structure MFI qui ont comme formule chimique approchée après calcination exprimée sous forme oxyde :
$M_{2/n}O, Ga_2O_3, xSiO_2$
où x peut varier de 12 à 1000 et où M représente le ou les cations de compensation de valence n. Le point important est que ces solides contiennent, après l'étape de synthèse et également après l'étape d'élimination des composés organiques, de l'élément fluor. La teneur en fluor dans la zéolithe déterminée par analyse élémentaire est comprise pour les solides calcinés, c'est-à-dire ceux résultant de l'étape (c) décrite précédemment, entre 0,02 et 1,5% en poids, avantageusement entre 0,05 et 1,0% et de préférence entre 0,2 et 0,8%.

## TABLEAU 1

DIAGRAMME DE DIFFRACTION RX DES GALLOSILICATES B CALCINEES A
STRUCTURE MONOCLINIQUE SELON L'INVENTION

(les mesures de $d_{hkl}$ ont été arrêtées à des valeurs de l'ordre de
2,97 Å, il est néanmoins possible d'effectuer des mesures de dhkl
pour des valeurs de 2 thêta plus grandes)

| dhkl Å ($10^{-10}$ m) | I/Io | dhkl Å ($10^{-10}$ m) | I/Io |
|---|---|---|---|
| 11,00–11,15 | FF | 4,22–4,27 | f |
| 9,99– 9,86 | F | 4,04–4,09 | ff |
| 9,67– 9,79 | m | 3,97–4,02 | ff |
| 8,89– 9,00 | fff | 3,90–3,95 | fff |
| 7,95– 8,06 | fff | 3,82–3,87 | F |
| 7,35– 7,44 | fff | 3,79–3,84 | mF |
| 6,99– 7,09 | fff | 3,77–3,82 | mF |
| 6,62– 6,71 | f | 3,72–3,77 | m |
| 6,29– 6,37 | f | 3,70–3,75 | m |
| 5,92– 6,00 | mf | 3,69–3,73 | m |
| 5,85– 5,93 | f | 3,63–3,6 | ff |
| 5,67– 5,74 | f | 3,60–3,65 | ff |
| 5,63– 5,70 | f | 3,42–3,46 | fff |
| 5,52– 5,59 | f | 3,32–3,37 | fff |
| 5,32– 5,39 | ff | 3,28–3,32 | ff |
| 5,09– 5,15 | fff | 3,23–3,27 | fff |
| 4,98– 5,04 | f | 3,03–3,07 | ff |
| 4,93– 4,99 | f | 3,01–3,05 | ff |
| 4,57– 4,63 | ff | 2,96–3,00 | f |
| 4,32– 4,38 | ff | 2,93–2,97 | ff |

## TABLEAU 2

### DIAGRAMME DE DIFFRACTION RX DES GALLOSILICATES A CALCINEES A STRUCTURE ORTHORHOMBIQUE SELON L'INVENTION

| dhkl $(10^{-10}$ m) Å | I/Io | dhkl $(10^{-10}$ m) Å | I/Io |
|---|---|---|---|
| 11,03–11,18 | FF | 4,32–4,38 | ff |
| 9,90–10,03 | F | 4,22–4,28 | f |
| 9,65– 9,78 | m | 4,05–4,11 | fff |
| 8,88– 9,00 | fff | 3,97–4,02 | ff |
| 7,93– 8,03 | fff | 3,82–3,87 | F |
| 7,36– 7,45 | fff | 3,78–3,83 | mF |
| 6,99– 7,09 | fff | 3,72–3,77 | m |
| 6,63– 6,72 | ff | 3,69–3,74 | m |
| 6,30– 6,38 | f | 3,62–3,67 | m |
| 5,93– 6,01 | mf | 5,56–3,61 | fff |
| 5,89– 5,97 | f | 3,41–3,45 | ff |
| 5,65– 5,72 | f | 3,32–3,37 | ff |
| 5,52– 5,59 | f | 3,29–3,33 | ff |
| 5,32– 5,39 | ff | 3,23–3,27 | fff |
| 5,07– 5,14 | fff | 3,03–3,07 | ff |
| 4,97– 5,04 | ff | 2,96–3,00 | f |
| 4,93– 4,99 | ff | 2,94–2,97 | ff |
| 4,57– 4,63 | ff | | |

Les mesures de dhkl ont été arrêtées à des valeurs de l'ordre de 2,97 Å, il est néanmoins possible d'effectuer des mesures de dhkl pour des valeurs de 2 thêta plus grandes.

Les zéolithes du type gallosilicate de la présente invention possèdent des propriétés acides tout à fait différentes de celles des zéolithes de structure MFI classiques obtenues en milieu alcalin (US-A-3702886 (1972); DE-A-2755770 (1974) US-A-4554146 (1985) ; JP-A-190818 (1983) et JP-A-64813 (1983). C'est la présente de fluor et plus spécialement la procédure par laquelle le fluor est incorporé dans les solides qui sont responsables de ces propriétés acides particulières.

L'acidité particulière des solides résultant de l'introduction de fluor lors de la synthèse, est la particularité mise à profit pour préparer des catalyseurs d'aromatisation susceptibles par exemple d'aromatiser un hydrocarbure comme le propane et plus généralement une coupe de gaz léger $C_2$-$C_4$ en présence ou non d'oléfines et dont les propriétés acides sont d'un type nouveau.

La présente invention concerne donc un catalyseur du type gallosilicate caractérisé par la composition suivante exprimée en poids :

a) 0,01 à 99,49% d'une matrice choisie dans le groupe formé par l'alumine, la silice, la magnésie, une argile et toute combinaison d'au moins deux des composés précités et

b) 0,51 à 99,99% d'une zéolithe synthétisée en milieu fluorure de formule chimique approchée suivante : $M_{2/n}O$, $Ga_2O_3$, $xSiO_2$, où

M représente un proton et/ou un cation métallique,

n est la valence du cation,

x est un nombre compris entre 12 et 1000,

la zéolithe ayant une teneur en fluor comprise entre 0,02 et 1,5% en poids, le fluor étant incorporé lors de la synthèse, la dite zéolithe étant aussi caractérisée par un diagramme de diffraction X présenté dans les tableaux I ou II.

De façon plus précise après synthèse en milieu fluorure, le solide peut être soumis si besoin est, à un traitement de défluoration permettant d'ajuster ses propriétés acides.

La zéolithe synthétisée renfermant du fluor et de type gallosilicate de structure MFI, est caractérisée par:

— une teneur en fluor comprise entre 0,02% et 1,5% en poids,

— un rapport molaire $Si^{IV}/Ga^{III}$ au moins égal à 8.6,

— un diagramme de diffraction X choisi dans le groupe constitué par le diagramme du tableau I à structure monoclinique et le diagramme du tableau II à structure orthorhombique.

De préférence, la zéolithe selon l'invention possède un spectre infra-rouge possédant des bandes Si-OH (vers 3740 cm$^{-1}$) et GaOH (vers 3620 cm$^{-1}$) moins intenses que les gallosilicates de l'art antérieur ayant le même rapport Si/Ga.

Le traitement de défluoration est plus ou moins sévère suivant le niveau de défluoration désiré. Il consiste en un ou plusieurs traitements successifs du solide, à reflux dans une solution d'ammoniaque de normalité comprise entre environ 0,05 et 5 N et de préférence entre 0,1 et 3 N, pendant une durée comprise entre environ 0,5 et 5 heures et de préférence entre 1 et 4 heures avec un rapport v/p défini comme le volume de solution par rapport au poids de solide sec compris entre environ 5 et 50 cm$^3$g$^{-1}$ et de préférence entre 10 et 30 cm$^3$g$^{-1}$. Le solide, après chaque lavage est ensuite abondamment lavé à l'eau distillée et séché à l'étuve. Après ces traitements et suivant leur sévérité, la teneur en fluor du solide est comprise entre 0,9 et 0,01% en poids. Si on élimine, par des traitements répétés, sensiblement la totalité du fluor, on aboutit encore à des solides qui se distinguent en particulier par leur spectre IR dans la région 3800-3500 cm$^{-1}$ des zéolithes de structure MFI classiques de même rapport Si/Ga de charpente : les solides contenus dans le catalyseur selon l'invention possèdent une proportion plus importante de groupes Si-OH.

Le solide partiellement ou totalement défluoré peut être mélangé à une matrice généralement amorphe, par exemple à une poudre humide de gel d'alumine. Le mélange est ensuite mis en forme, par exemple par extrusion au travers d'une filière. La teneur en zéolithe du support ainsi obtenu est généralement comprise entre environ 0,5 et 99,99% et avantageusement comprise entre environ 40 et 90% poids. Elle est plus particulièrement comprise entre environ 60 et 85 en poids par rapport à l'ensemble zéolithe et matrice.

La teneur en matrice du catalyseur est avantageusement comprise entre environ 10 et 60% et de préférence entre environ 15 et 40% en poids. La mise en forme peut être réalisée avec d'autres matrices que l'alumine, telles que, par exemple la magnésie, la silice-alumine, les argiles naturelles (kaolin, bentonite), et par d'autres techniques que l'extrusion telles que le pastillage ou la dragéification.

La zéolithe, partiellement défluorée ou non, peut, avant ou après la mise en forme avec une matrice amorphe, être traitée en présence de vapeur d'eau à haute température. Ce traitement, déjà préconisé dans le cas des gallosilicates synthétisés en milieu alcalin (WO 84/03879), augmente avantageusement les propriétés catalytiques du solide pour la réaction d'aromatisation d'une coupe $C_2$-$C_4$. Ce traitement consiste en une calcination conduite sous un gaz (air ou gaz contenant de l'air ou un gaz inerte), ce gaz contenant de préférence entre 5% et 100% de vapeur d'eau, à une température située entre 400 et 900°C, de préférence entre 450 et 600°C.

L'effet de ce traitement cumulé avec la présence de fluor apporte au solide un nouveau type d'acidité, conduit à un catalyseur aux performances pour la réaction d'aromatisation des gaz légers, améliorées par rapport aux catalyseurs de l'art antérieur ne contenant pas de fluor.

Le catalyseur obtenu par les procédures précédentes est mis en oeuvre pour la réaction d'aromatisation de gaz légers, par exemple du propane et/ou d'un mélange $C_2$-$C_4$ en présence ou non d'oléfines. Cette réaction revêt un intérêt particulier car elle permet de valoriser des résidus d'opérations de raffinage en des produits à plus haute valeur ajouté (benzène, toluène, xylène) tout en contribuant à la production de quantités importantes d'hydrogène indispensable pour les procédés d'hydrotraitement par exemple.

La charge contenant du butane, et/ou du propane et/ou de l'éthane en présence ou non d'oléfines est mise en contact avec le catalyseur préparé suivant les procédures précédentes, à une température comprise entre 400 et 700°C, et plus particulièrement entre 500 et 600°C.

Les exemples qui suivent précisent l'invention sans toutefois en limiter la portée, ils sont donnés pour une charge constituée uniquement de propane mais sont facilement transposables à une charge plus complexe comprenant un mélange de gaz légers $C_2$-$C_4$ avec ou sans oléfines.

EXEMPLE N° 1. Préparation des zéolithes A et B entrant dans la composition du catalyseur selon l'invention.

On prépare deux zéolithes de structure MFI de rapports atomiques Si/Ga proches de 25 et 100 à partir de mélanges réactionnels composés de :
+ une source de silice de composition $SiO_2$ commercialisée sous le nom d'aérosil 130,
+ une source de gallium : solution de chlorure de gallium préparée par dissolution de gallium métallique dans l'acide chlorhydrique concentré, la concentration en GaIII de cette solution est de 0,72 mole/l,
— fluorure d'ammonium
— bromure de tétrapropylammonium (TPA Br),
— germes de cristallisation (cristaux broyés de zéosilites ou gallozeosilites).
Les compositions molaires des différents mélanges réactionnels ainsi préparés, ramenés à une mole de silice sont les suivants :
$1SiO_2$ ; $xGaCl_3$ ; 0,25 TPABr ; 0,5 $NH_4F$ ; 50 $H_2O$
où x est égal à $5.10^{-2}$ dans le cas de la zéolithe A et $10^{-2}$ pour la zéolithe B.
Les synthèses sont effectuées dans des autoclaves revêtus intérieurement de polytétrafluoroéthylène, les autoclaves sont portés à 200°C pendant 4 jours.
Après refroidissement, les solides obtenus sont séparés des eaux mères par filtration puis lavés. Les cristaux calcinés (550°C, 8 heures) de ces deux échantillons possèdent des diagrammes de diffraction des rayons X analogues à ceux présentés dans le tableau 1 dans le cas de la zéolithe A et tableau 2 pour la zéolithe B. Les dimensions des cristaux sont voisines de quelques micromètres.
Dans le tableau 3, nous avons reporté en fonction du paramètre x qui caractérise le mélange réactionnel initial, les pH initiaux et finaux du milieu de synthèse ainsi que les caractéristiques physicochimiques principales des solides obtenus. Les données de ce tableau montrent que les solides A et B contiennent des quantités appréciables de fluor même après l'étape de calcination à 550°C sous air.

## TABLEAU 3

|  | Zéolithe A | Zéolithe B |  |
| --- | --- | --- | --- |
| x | $5.10^{-2}$ | $10^{-2}$ | 1 |
| pH initial | 2 | 7 |  |
| pH final | 2 | 5 |  |
| rapport molaire | 23,8 | 93,0 |  |
| % pds F⁻ avant calcination | 0,62 | 0,88 | 2 |
| % pds F⁻ après calcination à 550°C sous air | 0,3 | 0,2 |  |
| résultat des diagrammes de DX | orthorhombique (tableau 1) | monoclinique (tableau 2) |  |

(1) caractéristiques du mélange réactionnel

(2) caractéristiques des gallosilicates de structure MFI obtenus

EP 0 351 311 B1

<u>EXEMPLE N° 2</u>. Catalyseurs A1 et B1 conformes à l'invention.

Les zéolithes A et B de l'exemple 1 sont mises en forme par extrusion avec un liant de type aluminique à raison de 20% en poids de liant pour 80% en poids de zéolithe. Les solides obtenus référencés respectivement A1 et B1 sont calcinés à 600°C pendant 2 heures.

Ces deux catalyseurs sont testés en aromatisation du propane à 550°C et à pression atmosphérique. Le propane est dilué dans de l'argon à raison de 20% de $C_3H_8$ pour 80% d'Ar. Les performances catalytiques sont reportées dans le tableau 4.

Elles sont définies par :

```
PPH = vitesse massique horaire = débit horaire massique/masse de
                                    de C₃H₈          zéolithe
(conversion du) = (masse de propane)-(masse des )/(masse de propane
propane (% pds)     de la charge        produits de  de la charge
                                        la recette
(sélectivité en un) = 100 x (masse de Pi dans la recette)
  produit Pi(% pds)         (masse de propane) - (masse des produits
                            de la charge            de la recette
(rendement en un produit) =  masse du produit Pi dans la recette
  Pi (% poids)              masse de propane de la charge
```

Sont désignés par produits les hydrocarbures de la recette autres que le propane.

<u>EXEMPLE N° 3</u>. La préparation d'un catalyseur à partir de la zéolithe A défluorée de la présente invention, montre l'importance du fluor sur les propriétés catalytiques en aromatisation du propane.

On utilise comme zéolithe de départ la zéolithe A de l'exemple 1. La teneur en fluor de 0,3% après décomposition des cations structurants est ramenée à 0% par défluoration en milieu ammoniacal suivant le protocole décrit ci-après :

On fait subir à la zéolithe 3 cycles :
— solution $NH_4OH$ de concentration 0,2 N à 140°C pendant 4 heures
— filtration et lavage à l'eau distillée
— séchage à l'étuve à 150°C.

Après ce traitement on obtient un solide dont la cristallinité et le rapport Si/Ga ne sont pas altérés mais dont la teneur en fluor est en deça de la limite de détection de notre méthode de dosage qui est de 0,02% F (pds).

Le solide ainsi obtenu est mis en forme suivant les conditions de l'exemple 2 et est référencé A2. Testé en aromatisation du propane selon les conditions décrites dans l'exemple 2, on constate que les performances catalytiques de A2 reportées dans le tableau 4 sont inférieures au catalyseur A1 que ce soit du point de vue de la sélectivité en aromatiques mais surtout de l'activité catalytique.

<u>EXEMPLE N° 4</u>. Cet exemple nous montre l'intérêt d'un prétraitement à haute température en présence de vapeur d'eau des catalyseurs de la présente invention pour l'aromatisation du propane.

On utilise comme solide de départ le catalyseur B1 de l'exemple 2. Avant le test catalytique en aromatisation du propane, le catalyseur est prétraité in situ selon le protocole suivant :
— vitesse de montée en température 10°C/mn
— débit d'air 3 l $h^{-1}{}_g^{-1}$
— injection d'eau à 400°C et débit 2,25 cm³ $h^{-1}{}_g^{-1}$ d'eau liquide soit une teneur molaire en vapeur d'eau de 50%,
— température finale 500°C et palier de 30 mn à cette température.

<div align="center">8</div>

Le solide obtenu est référencé B2.

Le test catalytique dont les résultats sont présentés dans le tableau 4 montre que par rapport au solide B1 de départ l'activité catalytique est sensiblement augmenté mais surtout que la sélectivité en aromatique se trouve être plus élevée.

EXEMPLE N° 5. Catalyseur de comparaison C1.

La zéolithe C est une zéolithe de structure MFI synthétisée en milieu basique conventionnel dont la description figure dans le brevet US-A-4554146. Le solide obtenu a un rapport $Si^{IV}/Ga^{III}$ de 62,0 et ne contient pas du tout de fluor après synthèse. Mis en forme et testé en aromatisation du propane suivant les conditions de l'exemple 2, ce catalyseur référencé C1 s'avère être à la fois moins actif et moins sélectif en aromatiques que les catalyseurs de la présente invention. Ses performances catalytiques sont proches du catalyseur A1 qui est obtenu à partir d'une zéolithe selon la présente invention mais défluorée.

EXEMPLE N° 6. Catalyseur de comparaison C2.

Le catalyseur C1 de l'exemple 5 est prétraité suivant les conditions de l'exemple 4. Le solide obtenu référencé C2 est beaucoup moins actif que le solide B2 de la présente invention prétraité dans les mêmes conditions. Cet exemple montre l'influence du fluor sur les propriétés acides et donc l'activité des catalyseurs pour la réaction d'aromatisation du propane.

EXEMPLE N° 7. Catalyseur de comparaison C3.

La zéolithe C de l'exemple 5 est fluorée par un traitement à 450°C sous une atmosphère contenant $CHF_3$ pendant 4 heures. La teneur en fluor atteinte à l'issue de ce traitement est de 0,15% en poids. Ce solide est ensuite mis en forme et testé suivant les conditions de l'exemple 2. Les performances catalytiques du catalyseur C3 ainsi préparées sont reportées dans le tableau 4. Il apparaît clairement, qu'à teneur en fluor voisine des catalyseurs de la présente invention, les catalyseurs fluorés par traitement modificateur après synthèse ont des performances catalytiques médiocres.

TABLEAU 4

| Catalyseur | A1 | B1 | A2 | B2 | C1 | C2 | C3 |
|---|---|---|---|---|---|---|---|
| Si/Ga | 23,8 | 93,0 | 23,8 | 93,6 | 62,0 | 62,0 | 71,3 |
| % F | 0,3 | 0,2 | <0,02% | 0,2 | 0 | 0 | 0,15 |
| PPH | 0,8 | 0,8 | 0,8 | 0,8 | 0,8 | 0,8 | 0,8 |
| conversion propane | 83,0 | 45,6 | 70,1 | 62,1 | 45,0 | 51,4 | 12,9 |
| Rendement B, T, X | 44,1 | 22,0 | 21,7 | 35,4 | 14,5 | 18,2 | 0,5 |
| Sélectivité B,T,X | 54,0 | 49,1 | 31,2 | 57,0 | 32,3 | 33,5 | 4,1 |

## Revendications

1. Catalyseur renfermant en poids :

(a) 0,01 à 99,49% d'une matrice choisie dans le groupe formé par au moins l'alumine, la silice, la magnésie, une argile et

(b) 0,51 à 99,99% d'une zéolithe synthétisée en milieu fluorure et du type gallosilicate de structure MFI comprenant :

— une teneur en fluor comprise entre 0.02% et 1,5% en poids,

— un rapport molaire $Si^{IV}/Ga^{III}$ au moins égal à 8.6,

— un diagramme de diffraction X choisi dans le groupe constitué par le diagramme du tableau I à structure monoclinique et le diagramme du tableau II à structure orthorhombique, ci-dessous :

## TABLEAU 1

### DIAGRAMME DE DIFFRACTION RX DES GALLOSILICATES B CALCINEES A STRUCTURE MONOCLINIQUE SELON L'INVENTION

(les mesures de $d_{hkl}$ ont été arrêtées à des valeurs de l'ordre de 2,97 Å, il est néanmoins possible d'effectuer des mesures de dhkl pour des valeurs de 2 thêta plus grandes)

| dhkl Å $(10^{-10}m)$ | I/Io | dhkl Å $(10^{-10}m)$ | I/Io |
|---|---|---|---|
| 11,00-11,15 | FF | 4,22-4,27 | f |
| 9,99- 9,86 | F | 4,04-4,09 | ff |
| 9,67- 9,79 | m | 3,97-4,02 | ff |
| 8,89- 9,00 | fff | 3.90-3,95 | fff |
| 7,95- 8,06 | fff | 3,82-3,87 | F |
| 7,35- 7,44 | fff | 3,79-3,84 | mF |
| 6,99- 7,09 | fff | 3,77-3,82 | mF |
| 6,62- 6,71 | f | 3,72-3,77 | m |
| 6,29- 6,37 | f | 3,70-3,75 | m |
| 5,92- 6,00 | mf | 3,69-3,73 | m |
| 5,85- 5,93 | f | 3,63-3,6 | ff |
| 5,67- 5,74 | f | 3,60-3,65 | ff |
| 5,63- 5,70 | f | 3,42-3,46 | fff |
| 5,52- 5,59 | f | 3,32-3,37 | fff |
| 5,32- 5,39 | ff | 3,28-3,32 | ff |
| 5,09- 5,15 | fff | 3,23-3,27 | fff |
| 4,98- 5,04 | f | 3,03-3,07 | ff |
| 4,93- 4,99 | f | 3,01-3,05 | ff |
| 4,57- 4,63 | ff | 2,96-3,00 | f |
| 4,32- 4,38 | ff | 2,93-2,97 | ff |

## TABLEAU 2

### DIAGRAMME DE DIFFRACTION RX DES GALLOSILICATES A CALCINEES A STRUCTURE ORTHORHOMBIQUE SELON L'INVENTION

| dhkl $(10^{-10}m)$ Å | I/Io | dhkl $(10^{-10}m)$ Å | I/Io |
|---|---|---|---|
| 11,03-11,18 | FF | 4,32-4,38 | ff |
| 9,90-10,03 | F | 4,22-4,28 | f |
| 9,65- 9,78 | m | 4,05-4,11 | fff |
| 8,88- 9,00 | fff | 3,97-4,02 | ff |
| 7,93- 8,03 | fff | 3,82-3,87 | F |
| 7,36- 7,45 | fff | 3,78-3,83 | mF |
| 6,99- 7,09 | fff | 3,72-3,77 | m |
| 6,63- 6,72 | ff | 3,69-3,74 | m |
| 6,30- 6,38 | f | 3,62-3,67 | m |
| 5,93- 6,01 | mf | 5,56-3,61 | fff |
| 5,89- 5,97 | f | 3,41-3,45 | ff |
| 5,65- 5,72 | f | 3,32-3,37 | ff |
| 5,52- 5,59 | f | 3,29-3,33 | ff |
| 5,32- 5,39 | ff | 3,23-3,27 | fff |
| 5,07- 5,14 | fff | 3,03-3,07 | ff |
| 4,97- 5,04 | ff | 2,96-3,00 | f |
| 4,93- 4,99 | ff | 2,94-2,97 | ff |
| 4,57- 4,63 | ff | | |

Les mesures de dhkl ont été arrêtées à des valeurs de l'ordre de 2,97 Å, il est néanmoins possible d'effectuer des mesures de dhkl pour des valeurs de 2 thêta plus grandes.

2. Catalyseur selon la revendication 1 dans lequel la dite zéolithe possède un spectre infra-rouge possédant des bandes Si-OH (vers 3740 cm-1) et GaOH (vers 3620 cm-1) moins intenses que les gallosilicates de l'art antérieur ayant le même rapport Si/Ga.

3. Utilisation du catalyseur selon l'une des revendications 1 et 2 en aromatisation de gaz légers comportant 2 à 4 atomes de carbone par molécule.

## Patentansprüche

1. Katalysator, der in Gew.-% enthält :

(a) 0,01 bis 99,49% einer Matrix, ausgewählt aus der Gruppe, die gebildet wird von Aluminiumoxid, Siliciumdioxid, Magnesiumoxid und/oder einem Ton, und

(b) 0,51 bis 99,99% eines Zeoliths, der in einem Fluoridmedium synthetisiert worden ist, vom Gallosilikat-Typ mit MFI-Struktur, der aufweist

— einen Fluorgehalt zwischen 0,02 und 1,5 Gew.-%,

— ein Molverhältnis $Si^{IV}/Ga^{III}$ von mindestens 8,6,

— ein Röntgonbeugungsdiagramm, ausgewählt aus der Gruppe, die besteht aus dem Diagramm der nachstehenden Tabelle I mit monokliner Struktur und dem Diagramm der nachstehenden Tabelle II mit orthorhombischer Struktur :

## Tabelle I

Röntgenbeugungsdiagramm der calcinierten erfindungsgemäßen Gallosilikate mit monokliner Struktur

(die Messungen von $d_{hkl}$ wurden gestoppt bei Werten in der Größenordnung von 2,97 Å, es ist jedoch möglich, die Messungen von $d_{hkl}$ für größere Werte von $2\theta$ durchzuführen)

| $d_{hkl} Å$ $(10^{-10}m)$ | I/Io | $d_{hkl} Å$ $(10^{-10}m)$ | I/Io |
|---|---|---|---|
| 11.00-11.15 | FF | 4.22-4.27 | f |
| 9.89-9.96 | F | 4.04-4.09 | ff |
| 9.67-9.78 | m | 3.97-4.02 | ff |
| 8.89-9.00 | m | 3.90-3.96 | m |
| 7.95-8.06 | fff | 3.82-3.87 | F |
| 7.35-7.44 | fff | 3.79-3.84 | mF |
| 6.99-7.09 | fff | 3.77-3.82 | mF |
| 6.62-6.71 | f | 3.72-3.77 | m |
| 6.29-6.37 | f | 3.70-3.75 | m |
| 5.92-6.00 | mf | 3.69-3.73 | m |
| 5.85-5.93 | f | 3.53-3.6 | ff |
| 5.67-5.74 | f | 3.60-3.65 | ff |
| 5.63-5.70 | f | 3.42-3.46 | fff |
| 5.52-5.59 | f | 3.32-3.37 | fff |
| 5.32-5.39 | ff | 3.28-3.32 | ff |
| 5.09-5.15 | fff | 3.23-3.27 | fff |
| 4.98-5.04 | f | 3.03-3.07 | ff |
| 4.93-4.99 | f | 3.01-3.05 | ff |
| 4.57-4.63 | ff | 2.96-3.00 | f |
| 4.32-4.38 | ff | 2.93-2.97 | ff |

## Tabelle II

Röntgenbeugungsdiagramm der calcinierten erfindungsgemäßen Gallosilikate mit orthorhombischer Struktur

| $d_{hkl} (10^{-10}m)$ Å | I/Io | $d_{hkl}$ $(10^{-10}m)$ Å | I/Io |
|---|---|---|---|
| 11.03-11.15 | FF | 4.32-4.08 | ff |
| 9.90-10.03 | F | 4.22-4.28 | f |
| 9.65-9.76 | m | 4.05-4.11 | fff |
| 8.88-9.00 | fff | 3.97-4.02 | ff |
| 7.93-8.03 | fff | 3.82-3.87 | F |
| 7.36-7.45 | fff | 3.78-3.83 | mF |
| 6.99-7.09 | fff | 3.72-3.77 | m |
| 6.63-6.72 | ff | 3.69-3.74 | m |
| 6.30-6.38 | f | 3.62-3.67 | m |
| 5.93-6.01 | mf | 3.56-3.61 | fff |
| 5.59-5.67 | f | 3.41-3.46 | ff |
| 5.65-5.72 | f | 3.32-3.37 | ff |
| 5.52-5.59 | f | 3.28-3.33 | ff |
| 5.33-5.39 | ff | 3.23-3.27 | ff |
| 5.07-5.14 | fff | 3.03-3.07 | ff |
| 4.97-5.04 | ff | 2.96-3.00 | f |
| 4.83-4.89 | ff | 2.94-2.97 | ff |
| 4.57-4.63 | ff | | |

Die Messungen von $d_{hkl}$ wurden bei Werten in der Größenordnung von 2,97 Å gestoppt, es ist jedoch möglich, die Messungen von $d_{hkl}$ für größere Werte von $2\theta$ durchzuführen

2. Katalysator nach Anspruch 1, in dem der Zeolith ein IR-Spektrum hat, das Si-OH-Banden (bei etwa 3740 cm⁻¹) und GaOH-Banden (bei etwa 3620 cm⁻¹) aufweist, die weniger stark sind als bei den bekannten Gallosilikaten mit dem gleichen Si/Ga-Verhältnis.

3. Verwendung des Katalysators nach einem der Ansprüche 1 und 2 zur Aromatisierung von leichten Gasen, die 2 bis 4 Kohlenstoffatome pro Molekül enthalten.

## Claims

1. Catalyst containing by weight :

(a) 0.01 to 99.49% of a matrix chosen from the group formed by at least alumina, silica, magnesia, a clay and

(b) 0.51 to 99.99% of a zeolite synthesized in fluorine medium and of a gallosilicate type having a MFI structure comprising :

— a fluorine content ranging from 0.02 to 1.5% by weight,

— a $Si^{IV}/Ga^{III}$ molar ratio at least equal to 8.6,

— an X-ray diffraction diagram chosen from the group comprised of the diagram in table I having a monoclinical structure and the diagram in table II having an orthorhombic structure, herebelow

## TABLE 1

X-RAY DIFFRACTION DIAGRAM OF CALCINED GALLOSILICATES B
HAVING A MONOCLINICAL STRUCTURE ACCORDING TO THE INVENTION

(measurements of dhkl were stopped at values in the order of
2.97 Å, it is nonetheless possible to carry out measurements
of $d_{hkl}$ for higher 2 theta values)

| dhkl Å ($10^{-10}$ m) | I/Io | dhkl Å ($10^{-10}$ m) | I/Io |
|---|---|---|---|
| 11.00–11.15 | FF | 4.22–4.27 | f |
| 9.99– 9.86 | F | 4.04–4.09 | ff |
| 9.67– 9.79 | m | 3.97–4.02 | ff |
| 8.89– 9.00 | fff | 3.90–3.95 | fff |
| 7.95– 8.06 | fff | 3.82–3.87 | F |
| 7.35– 7.44 | fff | 3.79–3.84 | mF |
| 6.99– 7.09 | fff | 3.77–3.82 | mF |
| 6.62– 6.71 | f | 3.72–3.77 | m |
| 6.29– 6.37 | f | 3.70–3.75 | m |
| 5.92– 6.00 | mf | 3.69–3.73 | m |
| 5.85– 5.93 | f | 3.63–3.6 | ff |
| 5.67– 5.74 | f | 3.60–3.65 | ff |
| 5.63– 5.70 | f | 3.42–3.46 | fff |
| 5.52– 5.59 | f | 3.32–3.37 | fff |
| 5.32– 5.39 | ff | 3.28–3.32 | ff |
| 5.09– 5.15 | fff | 3.23–3.27 | fff |
| 4.98– 5.04 | f | 3.03–3.07 | ff |
| 4.93– 4.99 | f | 3.01–3.05 | ff |
| 4.57– 4.63 | ff | 2.96–3.00 | f |
| 4.32– 4.38 | ff | 2.93–2.97 | ff |

## TABLE 2

## X-RAY DIFFRACTION DIAGRAM OF CALCINED GALLOSILICATES A
## HAVING AN ORTHORHOMBIC STRUCTURE ACCORDING TO THE INVENTION

| dhkl ($10^{-10}$ m) Å | I/Io | dhkl ($10^{-10}$ m) Å | I/Io |
|---|---|---|---|
| 11.03–11.18 | FF | 4.32–4.38 | ff |
| 9.90–10.03 | F | 4.22–4.28 | f |
| 9.65– 9.78 | m | 4.05–4.11 | fff |
| 8.88– 9.00 | fff | 3.97–4.02 | ff |
| 7.93– 8.03 | fff | 3.82–3.87 | F |
| 7.36– 7.45 | fff | 3.78–3.83 | mF |
| 6.99– 7.09 | fff | 3.72–3.77 | m |
| 6.63– 6.72 | ff | 3.69–3.74 | m |
| 6.30– 6.38 | f | 3.62–3.67 | m |
| 5.93– 6.01 | mf | 3.56–3.61 | fff |
| 5.89– 5.97 | f | 3.41–3.45 | ff |
| 5.65– 5.72 | f | 3.32–3.37 | ff |
| 5.52– 5.59 | f | 3.29–3.33 | ff |
| 5.32– 5.39 | ff | 3.23–3.27 | fff |
| 5.07– 5.14 | fff | 3.03–3.07 | ff |
| 4.97– 5.04 | ff | 2.96–3.00 | f |
| 4.93– 4.99 | ff | 2.94–2.97 | ff |
| 4.57– 4.63 | ff | | |

Measurements of dhkl were stopped at values in the order of 2.97 A, it is nonetheless possible to carry out measurements of dhkl for higher 2 theta values.

2. Catalyst according to claim 1 wherein said zeolite has an infrared spectrum with Si-OH bands (around 3740 cm⁻¹) and GaOH bands (around 3620 cm⁻¹) that are less intense than the gallosilicates of the prior art having the same Si/Ga ratio.

3. Use of catalyst according to one of claims 1 and 2 in the aromatization of a light-gas cut having 2 to 4 carbon atoms per molecule.